# FASCICULE DE BREVET EUROPEEN

(11) **EP 2 508 220 B1**
(45) Date de publication et mention de la délivrance du brevet: **14.12.2016**
(21) Numéro de dépôt: 12160382.3
(22) Date de dépôt: 20.03.2012
(51) Int. Cl.: A61M 16/04, A61M 16/12, A61M 16/08

(54) **Dispositif d'assistance respiratoire**
Vorrichtung zur Atmungsunterstützung
Assisted breathing device

(30) Priorité: 06.04.2011 FR 1101035
(43) Date de publication de la demande: 10.10.2012
(73) Titulaire: Boussignac, Georges, 92160 Antony (FR)
(72) Inventeur: Boussignac, Georges, 92160 Antony (FR)
(74) Mandataire: Jacobacci Coralis Harle

(56) Documents cités:
- EP-A1- 0 092 618
- EP-A1- 0 640 355
- EP-A1- 0 978 291
- EP-A1- 2 044 969
- EP-A1- 2 228 088
- WO-A1-2007/071407
- WO-A2-2009/077667
- US-A- 4 231 365
- US-A1- 2007 240 722

## Description

La présente invention concerne un dispositif d'assistance respiratoire, apte à être avantageusement utilisé comme dispositif de respiration artificielle pendant la réanimation d'une personne en état d'arrêt cardiaque.

Notamment par la demande de brevet WO2009/077667, on connaît déjà un dispositif d'assistance respiratoire tubulaire pour des patients dont la respiration, bien que se produisant spontanément, est insuffisante. Un tel dispositif d'assistance respiratoire connu comporte :
- un tube qui forme un canal principal et qui est destiné à être relié, par son extrémité distale, à une voie respiratoire d'un patient pour que ledit canal principal relie à l'extérieur le système respiratoire de celui-ci ;
- des canaux auxiliaires périphériques reliés à une source de gaz respiratoire, pour pouvoir insuffler du gaz respiratoire dans le système respiratoire du patient, et qui débouchent, par leur extrémité distale, dans le canal principal ;
- des moyens pour défléchir, vers l'intérieur dudit canal principal, le gaz respiratoire injecté par les canaux auxiliaires ; et
- des moyens d'étanchéité, entourant ledit tube, aptes à assurer, au niveau du pharynx du patient, une communication fluidique étanche entre la trachée du patient et le canal principal.

Ainsi, pour obtenir une ventilation en gaz respiratoire satisfaisante d'un patient, il suffit d'introduire l'extrémité distale du dispositif précité jusqu'au pharynx de celui (et non jusqu'à la carène à travers le larynx et la trachée) pour assurer l'assistance respiratoire désirée.

Par ailleurs, l'expérience a montré que ce dispositif pour patient à respiration spontanée peut être utilisé avec succès comme dispositif de respiration artificielle (et non plus seulement comme dispositif d'assistance respiratoire) sur des personnes en état d'arrêt cardiaque en cours de réanimation par compressions et décompressions thoraciques alternées de leur cage thoracique, les jets de gaz respiratoire des canaux auxiliaires en continu favorisant la reprise de l'inspiration et de la circulation sanguine.

Toutefois, le Demandeur a remarqué que ledit gaz respiratoire, introduit en continu dans les poumons de la personne en état d'arrêt cardiaque, engendre dans ceux-ci, à la fin d'une compression et au début de la décompression suivante, une pression résiduelle positive, qui se maintient pendant une partie de ladite décompression, avant de disparaître et d'être remplacée par une pression négative engendrée par la décompression. Une telle pression résiduelle positive, d'une part, forme un obstacle à l'aspiration d'air extérieur à travers ledit élément tubulaire et, d'autre part, est entretenue par ledit air extérieur aspiré. Il en résulte que, pendant une partie importante de chaque décompression, les poumons de ladite personne aspirent mal l'air extérieur et que la circulation sanguine (notamment le retour veineux) n'est pas assurée de façon satisfaisante aux extrémités (tête, bras, jambes) de ladite personne.

En outre, l'application de compressions et décompressions sur la cage thoracique de la personne en cours de réanimation provoque fréquemment une perte d'étanchéité au niveau des moyens d'étanchéité, qui s'avère problématique, notamment en cas de reflux gastrique depuis l'oesophage. En effet, dans ce dernier cas, le reflux peut s'écouler dans la trachée, ou bien encore être introduit dans le canal principal du dispositif au risque d'entraîner son obstruction.

L'objet de la présente invention est donc de perfectionner le dispositif connu, rappelé ci-dessus, en supprimant les inconvénients précités.

A cette fin, selon l'invention, le dispositif d'assistance respiratoire comportant :
- un tube principal qui forme un canal principal et qui est destiné à être relié, par son extrémité distale, à une voie respiratoire d'un patient pour que ledit canal principal relie à l'extérieur le système respiratoire dudit patient ;
- au moins un canal auxiliaire périphérique qui est relié à une source de gaz respiratoire, pour pouvoir insuffler du gaz respiratoire dans ledit système respiratoire, et qui débouche, par son extrémité distale, dans une zone de déflexion ledit canal principal ;
- des moyens dans la zone de déflexion pour défléchir, vers l'intérieur dudit canal principal, le gaz respiratoire injecté par ledit canal auxiliaire ; et
- des moyens d'étanchéité, entourant ledit tube principal, aptes à assurer, au niveau du pharynx du patient, une communication fluidique étanche entre la trachée du patient et ledit canal principal du dispositif, est remarquable en ce qu'il comporte en outre un tube secondaire, formant un canal secondaire, qui s'étend à l'intérieur du tube principal, sur au moins une partie de sa longueur, et dont les extrémités proximale et distale sont destinées respectivement à être disposée à l'extérieur de la bouche du patient et à être reliée à l'oesophage de ce dernier.

Ainsi, grâce à l'invention, le tube secondaire provoque une restriction du canal principal, ce qui augmente la résistance s'exerçant sur l'écoulement gazeux traversant le canal principal et engendre, lors d'une compression de la cage thoracique d'une personne en cours de réanimation, une augmentation de pression (pression positive) à l'intérieur des poumons, l'air chassé de ces derniers s'échappant plus difficilement qu'en l'absence de tube secondaire.

Inversement, lors d'une décompression, la pression baisse davantage (pression négative) dans les poumons qu'avec un dispositif de respiration artificielle connu dépourvu de tube secondaire. Le freinage de l'entrée d'air extérieur, engendré par la restriction de diamètre, permet une aspiration progressive et contrôlée de l'air extérieur en direction des poumons de la personne, ce qui entraîne la disparition, au début de la décompression, de la pression résiduelle positive due aux jets de gaz respiratoire.

La pression résiduelle positive disparaît rapidement sous l'action de la décompression, pendant l'entrée progressive de l'air extérieur aspiré. La pression résiduelle positive ne constitue donc plus un obstacle à l'aspiration d'air extérieur et à la circulation sanguine de la personne en état d'arrêt cardiaque.

La variation de pression intrathoracique entre une compression et une décompression, obtenue selon l'invention, est étendue en comparaison des variations de pression intrathoracique observées sur des personnes en cours de réanimation équipées d'un dispositif d'assistance respiratoire connu, par exemple du type de celui décrit par la demande de brevet WO2009/077667. La surface d'échange gazeux est ainsi augmentée et le retour veineux amélioré.

En outre, le tube secondaire permet de réaliser une évacuation de liquide gastrique, ou encore une vidange de l'estomac le cas échéant, ce qui évite, en cas de défaut d'étanchéité des moyens d'étanchéité, un rejet dans la trachée ou une obstruction du canal principal du dispositif.

Il est à noter que le dispositif d'assistance respiratoire de l'invention peut être relié, de façon amovible, à un autre dispositif médical.

Les tubes principal et secondaire sont de préférence concentriques sur au moins une partie de la longueur dudit tube principal.

Le tube secondaire peut également être monté amovible par rapport audit dispositif d'assistance respiratoire, de manière à pouvoir être inséré et/ou retiré du canal principal selon l'utilisation désirée.

De préférence encore, lesdits moyens d'étanchéité sont conformés de telle façon à assurer une communication fluidique étanche entre l'oesophage dudit patient et ledit canal secondaire.

Dans une forme de réalisation conforme à la présente invention, lesdits moyens d'étanchéité se présentent sous la forme d'un bourrelet gonflable porté par la périphérie évasée et tronquée en biais d'une pièce en forme de trompette qui est rapportée à l'extrémité distale du tube principal.

De préférence, selon cette forme de réalisation :
- ledit tube secondaire traverse, de façon étanche, ledit bourrelet gonflable et se prolonge à l'extérieur de celui-ci ; et
- lesdits moyens d'étanchéité comportent en outre un ballonnet auxiliaire gonflable qui entoure ledit tube secondaire, sur sa portion se prolongeant au-delà du bourrelet gonflable, et qui est apte à assurer une communication fluidique étanche entre l'oesophage dudit patient et ledit canal secondaire.

Dans une autre forme de réalisation conforme à l'invention :
- au moins un orifice de communication traversant est ménagé dans la paroi latérale du tube principal, en aval des moyens de déflexion, pour permettre la communication fluidique, au niveau du pharynx du patient, entre la trachée de celui-ci et le canal principal ; et
- l'extrémité distale fermée du tube principal est traversée, de façon étanche, par le tube secondaire.

Selon cette autre forme de réalisation, les moyens d'étanchéité se présentent avantageusement sous la forme de deux ballonnets gonflables distincts de forme annulaire, dont l'un d'entre eux entoure l'extrémité distale du tube principal et l'autre entoure le tube principal de telle façon que ledit orifice de communication soit agencé entre les deux ballonnets.

Quelle que soit la forme de réalisation considérée, ledit canal auxiliaire peut déboucher dans un voisinage de l'extrémité proximale du canal principal ou au voisinage de l'extrémité distale de ce dernier.
Les figures du dessin annexé feront bien comprendre comment l'invention peut être réalisée. Sur ces figures, des références identiques désignent des éléments semblables.
La figure 1 est une vue schématique et partielle, en coupe axiale, d'un exemple de réalisation du dispositif de l'invention.
Les figures 2 et 3 sont des coupes transversales, respectivement selon les lignes II-II et III-III de la figure 1.
La figure 4 est une vue schématique des moyens d'étanchéité du dispositif de l'invention, selon la flèche IV de la figure 1.
La figure 5 illustre schématiquement la mise en place du dispositif de l'invention de la figure 1.
La figure 6 est une vue schématique et partielle, en coupe axiale, d'un autre exemple de réalisation du dispositif de l'invention.

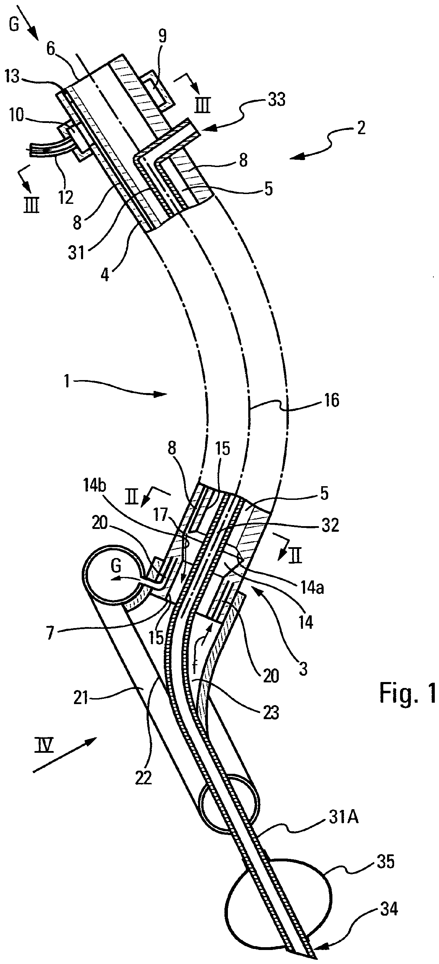

Sur la figure 1, on a représenté, schématiquement et à grande échelle, les seules extrémités proximale 2 et distale 3 d'un exemple de réalisation 1 du dispositif selon l'invention.

Le dispositif 1, conforme à l'invention, comporte un tube principal 4, souple (ou préformé pour s'adapter à la morphologie du patient) délimitant un canal principal 5 débouchant, par l'orifice 6, à l'extrémité proximale 2 et, par l'orifice 7, à l'extrémité distale 3.

Ainsi, le canal principal 5 est capable d'assurer le passage entre les orifices 6 et 7, dont l'un (l'orifice distal 7) est destiné à se trouver à l'intérieur des voies respiratoires d'un patient, et l'autre (l'orifice proximal 6) est destiné à se trouver à l'extérieur dudit patient. Cet orifice proximal 6 peut déboucher à l'air libre et, dans ce cas, le patient peut inspirer de l'air frais et expirer l'air vicié à travers le canal principal 5 (on pourrait également relier l'orifice 6 à une source de gaz respiratoire sous pression et prévoir un système de valves unidirectionnelles, pour que le patient inspire le gaz respiratoire de ladite source à travers ledit canal principal 5 et expire l'air vicié à l'air libre, également à travers ce canal principal).

Le diamètre du canal principal 5 est de l'ordre de quelques millimètres.

Par ailleurs, dans l'épaisseur de la paroi du tube principal 4, sont ménagés des canaux auxiliaires 8, s'étendant sur la presque totalité de la longueur du canal principal 5 et destinés à être reliés à une source de gaz respiratoire sous pression, comme cela est décrit ci-après.

La liaison à la source de gaz respiratoire sous pression peut être réalisée au moyen d'une bague 9, entourant de façon étanche le tube 4, du côté de l'extrémité proximale 2, et délimitant une chambre annulaire étanche 10 autour dudit tube. Les canaux auxiliaires 8 sont mis en communication avec la chambre 10, grâce à des arrachements locaux 11 de la paroi du tube 4, et ladite chambre 10 est reliée à ladite source de gaz respiratoire par un conduit 12. Bien entendu, les extrémités proximales des canaux 8 sont obturées, par exemple par des bouchons 13, introduits à partir de la face d'extrémité proximale du tube 4.

Les canaux auxiliaires 8 ont un diamètre plus petit que celui du canal principal 5. Le diamètre des canaux auxiliaires 8 est de préférence inférieur à 1 mm et, de façon avantageuse, il est de l'ordre de 400 à 800 microns. Du côté distal, les canaux auxiliaires 8 débouchent dans un évidement 14 de la paroi interne 15 du tube 4. L'évidement 14 est annulaire et centré sur l'axe 16 de l'extrémité distale 3. Il comporte une face 14a, sensiblement transversale ou légèrement inclinée de façon à constituer un évasement du canal principal 5, dans laquelle débouchent lesdits canaux auxiliaires 8 par leurs orifices 17, ainsi qu'une face 14b suivant la face 14a et convergeant en direction de l'axe 16.

Ainsi, lorsque les canaux auxiliaires 8 sont alimentés en gaz respiratoire sous pression à travers les éléments 9 à 1 2, les jets gazeux correspondants heurtent la face inclinée 14b, qui les défléchit en direction de l'axe 16 (voir la flèche sur la figure 1 à la sortie des orifices 17), engendrant à l'intérieur de l'extrémité distale 3 du canal principal 5 une zone de pression de forme oblongue prenant naissance auxdits orifices distaux 17 et s'allongeant en direction de l'orifice distal 7 le long de l'axe 16 de ladite extrémité distale 3. La section transversale de cette zone de pression décroît progressivement depuis l'évidement 14 vers l'orifice distal 7, ladite zone de pression s'écartant progressivement de la paroi interne 15 du tube 4 pour n'occuper que la partie centrale de l'extrémité distale 3 de ce dernier. En aval de la zone de pression, les jets de gaz respiratoire défléchis engendrent au voisinage de l'axe 16 une zone de dépression favorisant la circulation gazeuse à l'intérieur du canal principal 5, de l'orifice proximal vers l'orifice distal. On favorise ainsi l'inspiration du patient.

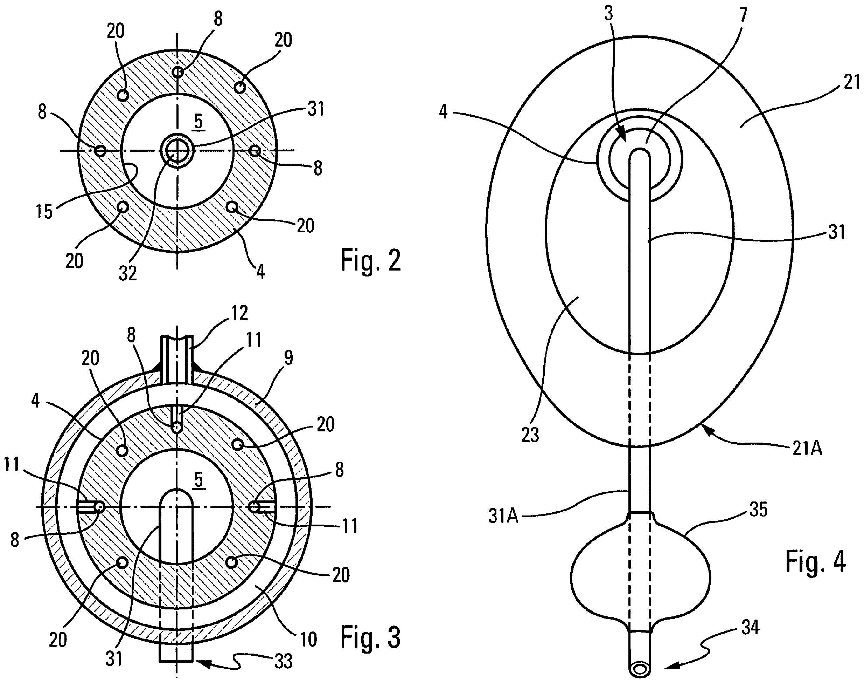

Comme le montrent les figures 2 et 3, les canaux auxiliaires 8 sont disposés régulièrement autour de l'axe du tube 4. Leur nombre est variable suivant les utilisations (adulte ou enfant), mais il est généralement compris entre trois et neuf.

Le tube 4 du dispositif selon l'invention peut être réalisé en toute matière déjà utilisée dans les sondes respiratoires, par exemple en un chlorure de polyvinyle, avec un éventuel revêtement de silicone.

Des canaux supplémentaires 20 sont prévus dans l'épaisseur dudit tube 4. Ces canaux 20 peuvent être utilisés à des fins différentes, telles qu'injection d'un médicament fluide, prise de pression, prélèvement d'échantillons gazeux (comme indiqué symboliquement par la flèche f se trouvant en regard d'un canal 20 à la partie inférieure de la figure 1) et, comme il sera décrit ci-après, gonflage de bourrelet d'étanchéité. On remarquera que, sur la figure 1, à des fins de simplification du dessin, on a montré un canal 8 et des portions de canaux 20, bien que ces canaux soient situés dans des plans différents (voir les figures 2 et 3).

En effet, comme le montrent les figures 1 et 4, à l'extrémité distale 3 du tube 4, est rapporté un bourrelet gonflable 21, de forme annulaire et porté à la périphérie 22 d'une pièce 23. La pièce 23 présente la forme au moins approximative d'une trompette et est emboîtée par sa petite extrémité sur l'extrémité distale 3 du tube 4. L'extrémité évasée de la pièce 23 est tronquée en biais, de façon que ladite périphérie 22 et le bourrelet gonflable 21 qu'elle porte, soient inclinés par rapport à l'axe 16 de l'extrémité distale 3. De la sorte, lorsque le dispositif 1 est introduit à l'état dégonflé dans un patient 25, à travers la bouche 26 et le pharynx 27 de celui-ci, le bourrelet 21 est apte, après gonflage par un gaz de gonflage G amené depuis une source (non représentée) jusqu'au dispositif 1, puis transmis au bourrelet 21 à travers un canal 20, à entourer la larynx 28 et à assurer une communication gazeuse au moins sensiblement étanche entre la trachée 29 du patient 25 et le canal principal 5 du dispositif 1 (voir la figue 5). Dans cette dernière position, le bourrelet 21 obture partiellement l'oesophage 30 par sa portion conformée 21 A.

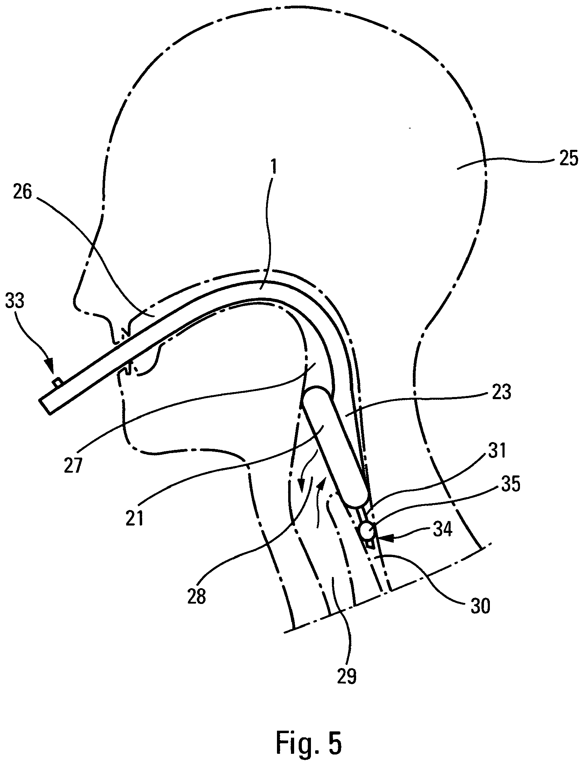

Comme le montre la figure 1, selon l'invention, le dispositif 1 comporte également un tube secondaire souple 31, formant un canal secondaire 32, qui s'étend à l'intérieur du tube principal 4, sur la quasi-totalité de sa longueur. L'extrémité proximale 33 est destinée à être disposée à l'extérieur de la bouche du patient, alors que l'extrémité distale 34 est, quant à elle, destinée à être reliée à l'oesophage 30 du patient comme l'illustre la figure 5.

Dans cet exemple, les tubes principal 4 et secondaire 31 sont concentriques, le canal principal 5 présentant un diamètre interne sensiblement supérieur au diamètre externe du tube secondaire 31.

En outre, comme l'illustrent les figures 1 et 4, le tube secondaire 31 traverse, de façon étanche, le bourrelet gonflable 21 et se prolonge à l'extérieur de celui-ci.

La portion 31 A du tube 31, qui s'étend au-delà du bourrelet 21, comporte un ballonnet gonflable auxiliaire 35, de forme annulaire, qui l'entoure. Ce dernier assure, une fois correctement positionné et gonflé, une communication fluidique étanche entre l'oesophage 30 du patient et le canal secondaire 32.

Un canal supplémentaire (non représenté sur les figures) est prévu dans l'épaisseur du tube secondaire 31 et débouche dans le ballonnet auxiliaire 35 pour permettre l'injection d'un gaz de gonflage dans ce dernier.

On peut ainsi, sans introduction du tube 4 dans la trachée 29, ventiler les poumons (non représentés) du patient 25 à l'aide d'un gaz respiratoire introduit dans le canal principal 5 par les canaux 8 et les moyens de déflexion 14b et permettre l'exhalation vers l'extérieur du gaz vicié sortant desdits poumons (voir les deux flèches sur la figure 5).

En outre, lorsque l'extrémité distale 34 du tube secondaire 31 est insérée dans l'oesophage 30 et que le ballonnet 35 est gonflé, on évite tout écoulement gastrique dans la trachée ou dans le pharynx, en cas de défaut d'étanchéité du bourrelet 21, le tube 31 permettant l'évacuation dudit écoulement.

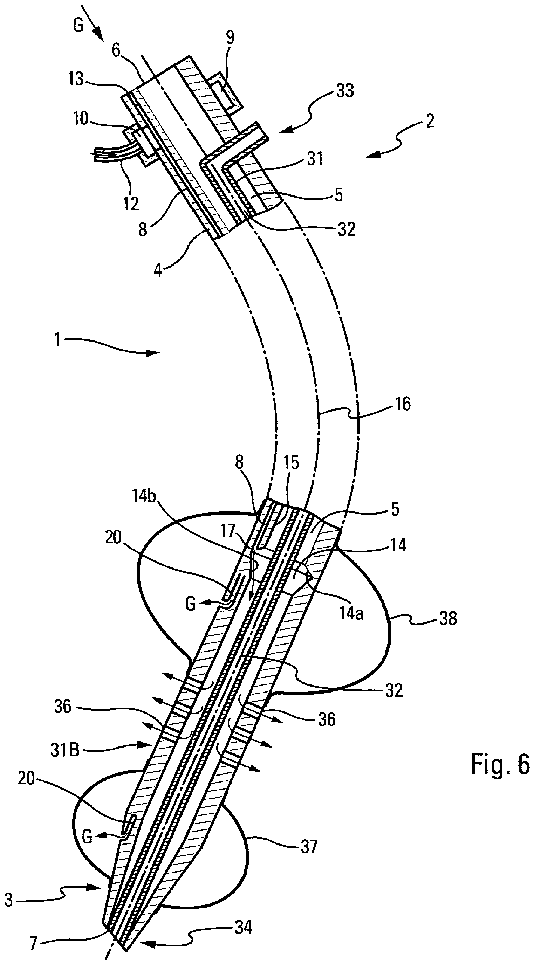

Sur la figure 6, on a représenté, en vue semblable à la figure 1, un autre exemple de réalisation du dispositif 1 de l'invention. Dans les exemples des figures 1 et 6, des références numériques identiques désignent des éléments semblables.

Comme le montre la figure 6, le tube principal 4 se prolonge, à son extrémité distale 3, au-delà de l'évidement 14 par une portion tubulaire 31B, centrée sur l'axe 16, dont les parois latérales convergent en direction de l'axe 16 et sont solidarisées à la paroi latérale externe du tube secondaire 31, à son extrémité distale. Autrement dit, dans cet exemple, l'extrémité distale 7 du canal principal 5 est fermée hermétiquement, seule l'extrémité distale 34 du canal secondaire 32 étant ouverte pour permettre une communication fluidique entre l'oesophage 30 et le canal 32.

Par ailleurs, plusieurs orifices de communication traversants 36 sont ménagés dans la paroi latérale du tube principal 4, en aval de l'évidement 14, pour permettre la communication fluidique, au niveau du pharynx 27 du patient, entre la trachée 29 de celui-ci et le canal principal 5.

Un premier ballonnet gonflable 37, de forme annulaire, entoure la paroi latérale du tube principal 4, à son extrémité distale. Ce ballonnet 37 est destiné à être introduit dans l'oesophage 30 du patient pour assurer une communication fluidique étanche entre ledit oesophage 30 et le canal secondaire 32.

Un second ballonnet gonflable 38, également de forme annulaire, entoure la paroi latérale du tube principal 4 de telle façon que les orifices de communication 36 soient tous intercalés entre les deux ballonnets 37 et 38. Ainsi faisant, une communication fluidique étanche, au niveau du pharynx 27 du patient, peut être obtenue entre la trachée 29 de celui-ci et le canal principal 5, une fois les deux ballonnets 37 et 38 gonflés.

Après avoir été introduit à l'état dégonflé à travers la bouche 26 et le pharynx 27 dans un patient 25, les ballonnets 37 et 38 en position sont gonflés par un gaz de gonflage G amené depuis une source (non représentée) jusqu'au dispositif 1, puis transmis auxdits ballonnets 37 et 38 à travers des canaux d'alimentation 20 (partiellement représentés).

## Revendications

1. Dispositif d'assistance respiratoire comportant :
- un tube principal (4) qui forme un canal principal (5) et qui est destiné à être relié, par son extrémité distale (3), à une voie respiratoire d'un patient (25) pour que ledit canal principal relie à l'extérieur le système respiratoire dudit patient ;
- au moins un canal auxiliaire périphérique (8) qui est relié à une source de gaz respiratoire, pour pouvoir insuffler du gaz respiratoire dans ledit système respiratoire, et qui débouche, par son extrémité distale, dans une zone de déflexion dans ledit canal principal (5) ;
- des moyens (14b) dans la zone, de déflexion pour défléchir, vers l'intérieur dudit canal principal (5), le gaz respiratoire injecté par ledit canal auxiliaire (8) ; et
- des moyens d'étanchéité (21; 37, 38), entourant ledit tube principal (4), aptes à assurer, au niveau du pharynx (27) du patient, une communication fluidique étanche entre la trachée (29) du patient et ledit canal principal (5),
**caractérisé en ce qu'**il comporte un tube secondaire (31), formant un canal secondaire (32), qui s'étend à l'intérieur du tube principal (4), sur au moins une partie de sa longueur, et dont les extrémités proximale (33) et distale (34) sont destinées respectivement à être disposée à l'extérieur de la bouche (25) du patient et à être reliée à l'oesophage (30) de ce dernier.

2. Dispositif selon la revendication 1, **caractérisé en ce que** les tubes principal (4) et secondaire (31) sont concentriques sur au moins une partie de la longueur dudit tube principal (4).

3. Dispositif selon l'une des revendications 1 ou 2, **caractérisé en ce que** lesdits moyens d'étanchéité (35, 37) sont conformés de telle façon à assurer une communication fluidique étanche entre l'oesophage (30) dudit patient et ledit canal secondaire (31).

4. Dispositif selon l'une des revendications 1 à 3, **caractérisé en ce que** le tube secondaire (31) est monté amovible par rapport audit dispositif (1).

5. Dispositif selon l'une des revendications 1 à 4, **caractérisé en ce que** lesdits moyens d'étanchéité se présentent sous la forme d'un bourrelet gonflable (21) porté par la périphérie évasée et tronquée en biais d'une pièce (23) en forme de trompette qui est rapportée à l'extrémité distale (3) du tube principal (4).

6. Dispositif selon la revendication 5, **caractérisé :**
- **en ce que** ledit tube secondaire (31) traverse, de façon étanche, ledit bourrelet gonflable (21) et se prolonge à l'extérieur de celui-ci ; et
- **en ce que** lesdits moyens d'étanchéité comportent en outre un ballonnet gonflable auxiliaire (35) qui entoure ledit tube secondaire (31), sur sa portion se prolongeant au-delà dudit bourrelet gonflable (21), et qui est apte à assurer une communication fluidique étanche entre l'oesophage (30) dudit patient et ledit canal secondaire (31).

7. Dispositif selon l'une des revendications 1 à 4, **caractérisé :**
- **en ce qu'**au moins un orifice de communication traversant (36) est ménagé dans la paroi latérale du tube principal (4), en aval des moyens de déflexion (14b), pour permettre la communication fluidique, au niveau du pharynx (27) du patient, entre la trachée (29) de celui-ci et le canal principal (4) ; et
- **en ce que** l'extrémité distale fermée (3) du tube principal (4) est traversée, de façon étanche, par le tube secondaire (31).

8. Dispositif selon la revendication 7, **caractérisé en ce que** les moyens d'étanchéité se présentent sous la forme de deux ballonnets gonflables distincts (37, 38) de forme annulaire, dont l'un d'entre eux entoure l'extrémité distale (3) du tube principal (4) et l'autre entoure le tube principal de telle façon que ledit orifice de communication (36) soit agencé entre les deux ballonnets (37, 38).

9. Dispositif selon l'une des revendications 1 à 8, **caractérisé en ce que** ledit canal auxiliaire (8) débouche dans un voisinage de l'extrémité proximale (2) dudit canal principal (4).

10. Dispositif selon l'une des revendications 1 à 8, **caractérisé en ce que** ledit canal auxiliaire (8) débouche au voisinage de l'extrémité distale (3) dudit canal principal (4).

## Patentansprüche

1. Vorrichtung zur Atmungsunterstützung mit
- einem Hauptrohr (4), das einen Hauptkanal (5) bildet und dazu bestimmt ist, mit seinem distalen Ende (3) mit einem Atemweg eines Patienten (25) verbunden zu werden, damit der Hauptkanal das Atmungssystem des Patienten nach draußen verbindet,
- wenigstens einem peripheren Hilfskanal (8), der mit einer Quelle für Beatmungsgas verbunden ist, zum Beatmungsgas in das Atmungssystem einblasen zu können, und der mit seinem distalen Ende in einen Ablenkbereich im Hauptkanal (5) mündet,
- Mitteln (14b) im Ablenkbereich, um das durch den Hilfskanal (8) eingeleitete Beatmungsgas zum Inneren des Hauptkanals (5) hin abzulenken, und
- Dichtmitteln (21; 37, 38), die das Hauptrohr (4) umgeben und geeignet sind, im Bereich des Rachens (27) des Patienten eine dichte Fluidverbindung zwischen der Luftröhre (29) des Patienten und dem Hauptkanal (5) herzustellen,
**dadurch gekennzeichnet, daß** sie ein Sekundärrohr (31) aufweist, das einen Sekundärkanal (32) bildet, der sich über mindestens einen Teil seiner Länge im Inneren des Hauptrohrs (4) erstreckt und dessen proximales Ende (33) und distales (34) Ende dazu bestimmt sind, außerhalb des Munds des Patienten (25) angeordnet bzw. mit der Luftröhre (30) des letzteren verbunden zu sein.

2. Vorrichtung gemäß Anspruch 1, **dadurch gekennzeichnet, daß** das Hauptrohr (4) und das Sekundärrohr (31) mindestens auf einem Teil der Länge des Hauptrohrs (4) konzentrisch sind.

3. Vorrichtung gemäß einem der Ansprüche 1 oder 2, **dadurch gekennzeichnet, daß** die Dichtmittel (35, 37) so ausgebildet sind, daß sie eine dichte Fluidverbindung zwischen der Luftröhre (30) des Patienten und dem Sekundärkanal (31) sicherstellen.

4. Vorrichtung gemäß einem der Ansprüche 1 bis 3, **dadurch gekennzeichnet, daß** das Sekundärrohr (31) an der Vorrichtung (1) abnehmbar angebracht ist.

5. Vorrichtung gemäß einem der Ansprüche 1 bis 4, **dadurch gekennzeichnet, daß** die Dichtmittel in Form eines aufblasbaren Wulsts (21) vorliegen, der vom aufgeweiteten und schräg angeschnittenen Rand eines trompetenförmigen Teils (23) getragen wird, das am distalen Ende (3) des Hauptrohrs (4) angetragen ist.

6. Vorrichtung gemäß Anspruch 5, **dadurch gekennzeichnet, daß**
- das Sekundärrohr (31) in abgedichteter Weise den aufblasbaren Wulst (21) durchquert und sich außerhalb desselben fortsetzt und daß
- die Dichtmittel außerdem einen aufblasbaren Hilfsbalg (35) aufweisen, der auf seinem sich außerhalb des aufblasbaren Wulsts (21) fortsetzenden Abschnitt das Sekundärrohr (31) umgibt und der geeignet ist, eine dichte Fluidverbindung zwischen der Luftröhre (30) des Patienten und dem Sekundärkanal (31) sicherstellen.

7. Vorrichtung gemäß einem der Ansprüche 1 bis 4, **dadurch gekennzeichnet, daß**
- in Fließrichtung nach den Ablenkmitteln (14b) wenigstens ein Verbindungsdurchgangsloch (36) in der Seitenwand des Hauptrohrs (4) eingebracht ist, um im Bereich des Rachens (27) des Patienten eine Fluidverbindung zwischen des Luftröhre (29) des letzteren und dem Hauptkanal (4) zu ermöglichen, und daß
- das geschlossene distale Ende (3) des Hauptrohrs (4) in abgedichteter Weise vom Sekundärrohr (31) durchquert wird.

8. Vorrichtung gemäß Anspruch 7, **dadurch gekennzeichnet, daß** die Dichtmittel in Form von zwei getrennten aufblasbaren ringförmigen Balgen (37, 38) vorliegen, von denen der eine das distale Ende (3) des Hauptrohrs (4) umgibt und der andere das Hauptrohr derart umgibt, daß das Verbindungsloch (36) zwischen den beiden Balgen (37, 38) angeordnet ist.

9. Vorrichtung gemäß einem der Ansprüche 1 bis 8, **dadurch gekennzeichnet, daß** der Hilfskanal (8) in eine Umgebung des proximalen Endes (2) des Hauptkanals (4) mündet.

10. Vorrichtung gemäß einem der Ansprüche 1 bis 8, dadurch gekennzeichet, daß der Hilfskanal (8) in eine Umgebung des distalen Endes (3) des Hauptkanals (4) mündet.

## Claims

1. A respiratory assistance device comprising:
- a main tube (4) forming a main channel (5) and configured for being connected, via its distal end (3), to a patient's (25) airway so that said main channel connects to the exterior the said patient's respiratory system;
- at least one peripheral auxiliary channel (8) being connected to a source of respiratory gas so as to blow respiratory gas in said respiratory system and opening up, via its distal end, in a deflection zone in said main channel (5);
- means (14b) in the deflection zone for deflecting, to the interior of said main channel (5), the respiratory gas injected by said auxiliary channel (8); and
- sealing means (21; 37, 38), surrounding said main tube (4), able to ensure, at the patient's pharynx (27) level, a sealed fluid communication between the patient's trachea (29) and said main channel (5),
**characterized in that** it comprises a secondary tube (31), forming a secondary channel (32), extending inside the main tube (4), on at least part of its length, and which the proximal (33) and distal (34) ends are configured respectively for being arranged outside the patient's mouth (25) and for being connected to the esophagus (30) of the latter.

2. Device according to claim 1, **characterized in that** the main (4) and secondary (31) tubes are concentric on at least part of the length of said main tube (4).

3. Device according to claim 1 or 2, **characterized in that** sealing means (35, 37) are shaped so as to ensure a sealed fluid communication between said patient's esophagus (30) and said secondary channel (31).

4. Device according to one of claims 1 to 3, **characterized in that** the secondary tube (31) is removably mounted with respect to said device (1).

5. Device according to one of claims 1 to 4, **characterized in that** said sealing means have the shape of an inflatable bead (21) supported by the flared and obliquely truncated periphery of a trumpet shaped part (23) that is assembled at the distal end (3) of the main tube (4).

6. Device according to claim 5, **characterized:**
- **in that** said secondary tube (31) tightly goes through said inflatable bead (21) and extends outside thereof; and
- **in that** said sealing means further comprise an inflatable auxiliary balloon (35) surrounding said secondary tube (31), on its portion extending beyond said inflatable bead (21), and configured to ensure a sealed fluid communication between said patient's esophagus (30) and said secondary channel (31).

7. Device according to one of claims 1 to 4, **characterized:**
- **in that** at least one communication through-hole (36) is arranged in the side wall of the main tube (4), downstream the deflection means (14b), so as to achieve the fluid communication, at the level of the patient's pharynx (27), between his/her trachea (29) and the main channel (4); and
- **in that** the closed distal end (3) of the main tube (4) is tightly crossed by the secondary tube (31).

8. Device according to claim 7, **characterized in that** the sealing means have the shape of two distinct ring-shaped inflatable balloons (37, 38), wherein one of them surrounds the distal end (3) of the main tube (4) and the other one surrounds the main tube so that said communication hole (36) is arranged between the two balloons (37, 38).

9. Device according to one of claims 1 to 8, **characterized in that** said auxiliary channel (8) opens up in a vicinity of the proximal end (2) of said main channel (4).

10. Device according to one of claims 1 to 8, **characterized in that** said auxiliary channel (8) opens up in the vicinity of the distal end (3) of said main channel (4).
